# EUROPEAN PATENT APPLICATION

(11) **EP 4 140 478 A1**
(43) Date of publication of application: **01.03.2023**
(21) Application number: 22191584.6
(22) Date of filing: 22.08.2022
(51) Int. Cl.: A61K 9/20, A61K 31/41, A61K 31/216

(54) **A SOLID PHARMACEUTICAL COMPOSITION COMPRISING SACUBITRIL AND VALSARTAN**

(30) Priority: 24.08.2021 TR 202113354
(71) Applicant: SANOVEL ILAC SANAYI VE TICARET A.S., Istanbul 34460 (TR)
(72) Inventor: KARABULUT, Serif, Istanbul (TR); ERDOGAN, Akif, Istanbul (TR); BILGEHAN ATAK, Fadime, Istanbul (TR); SUNEL, Fatih, Istanbul (TR)
(74) Representative: Sevinç, Erkan

(57) **Abstract**

The present invention relates to a solid pharmaceutical composition comprises sacubitril in the form of the free base or in the form of pharmaceutically acceptable salts, crystalline polymorph thereof and valsartan in the form of the free base or in the form of pharmaceutically acceptable salts, crystalline polymorph thereof and at least one pharmaceutically acceptable excipient wherein the amount of binder is 0.1% to 20.0% by weight of the total composition. Further, the present invention also relates

## Description

### Field of the Invention

The present invention relates to a solid pharmaceutical composition comprises sacubitril in the form of the free base or in the form of pharmaceutically acceptable salts, crystalline polymorph thereof and valsartan in the form of the free base or in the form of pharmaceutically acceptable salts, crystalline polymorph thereof and at least one pharmaceutically acceptable excipient wherein the amount of binder is 0.1% to 20.0% by weight of the total composition. Further, the present invention also relates to a simple, rapid, cost effective, time-saving and industrially convenient process.

### Background of the Invention

Sacubitril is an antihypertensive drug used in combination with valsartan. Its chemical designation is 4-(((2S,4R)-1-([1,1'-biphenyl]-4-yl)-5-ethoxy-4-methyl-5-oxopentan-2-yl)amino)-4-oxobutanoic acid with the following chemical structure of Formula I.

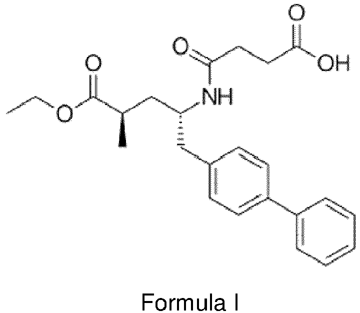

Valsartan is a specific angiotensin II receptor antagonist, which selectively and competitively blocks AT1-receptors and mediates the vasopressor and aldosterone effects of angiotensin II. Its chemical designation is N-(1-oxopentyl)-N-[[2'-(1H-tetrazol-5-yl)-[1,1'-biphenyl]-4-yl]methyl]-L-valine with the following chemical structure of Formula II.

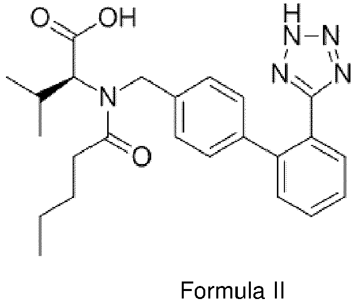

A complex comprising valsartan, which is an angiotensin receptor antagonist, and Sacubitril, which is a neurolysin inhibitor, has been approved by US Food and Drug Administration (FDA) under the trade name ENTRESTO^{®} by Novartis for the treatment of heart failure with reduced ejection fraction. Entresto^{®} is supplied for oral administration as immediate release film-coated tablets in three strengths: 24/26, 49/51 and 97/103 mg, containing respectively 24.3 mg sacubitril/25.7 mg valsartan, 48.6 mg sacubitril/51.4 mg valsartan and 97.2 mg sacubitril/102.8 mg valsartan.

US 8101659 and US 8404744 discloses a composition comprising Valsartan and Sacubitril which are administered in combination in 1:1 ratio.

US 8796331 discloses a method of treating hypertension and heart failure by administering a combination of Valsartan and Sacubitril administered in one unit dose form or in two separate unit dose forms.

Sacubitril and pharmaceutically acceptable salts thereof and valsartan and pharmaceutically acceptable salts thereof are solid but very hygroscopic substances. It is well known that the handling and the formulation of hygroscopic active ingredients into solid pharmaceutical formulations is difficult and requires extensive precautions. When water absorption occurs during manufacturing, the consequences include processing problems such as stickiness, clumping, poor release from punches, poor flow characteristics and poor compressibility. Furthermore, stability problems after storage are occurred.

Another problem is that valsartan has very poor solubility at pH below 5. Above pH 5, both sacubitril and valsartan exhibit high solubility in aqueous medium. The major limitation of the drug is its low solubility and permeability leading to poor bioavailability.

In prior art, low solubility of the active agents and stability problems has been approached in several ways. However, these studies in the prior art have been it strongly affects the dissolution profile of pharmaceutical compositions comprising valsartan and sacubitril, and therefore causes problems with the development of a pharmaceutical formulation with a specified dissolution profile. it is not the case that only the careful selection of excipients, binders, disintegrants and other ingredients customarily used in pharmaceutical compositions or reducing particle size of active agent is important for the development of a composition with favorable solubility characteristics.

There is thus still a need for a solid pharmaceutical composition comprises sacubitril in the form of the free base or in the form of pharmaceutically acceptable salts, crystalline polymorph thereof, valsartan in the form of the free base or in the form of pharmaceutically acceptable salts, crystalline polymorph thereof that provides a solid pharmaceutical composition having the desired stability, dissolution profile, hardness and compressibility, in another words the disadvantages seen in the active substance will able to overcome. The formulation has been developed by using standard techniques which is simple and cost-effective method.

### Detailed Description of the Invention

The main object of the present invention is to eliminate problems caused by active substances and bringing additional advantages to the relevant prior art.

Another object of the present invention is to provide a solid pharmaceutical composition comprising sacubitril in the form of the free base or in the form of pharmaceutically acceptable salts, crystalline polymorph thereof and valsartan in the form of the free base or in the form of pharmaceutically acceptable salts, crystalline polymorph thereof with high stability, having desired level of dissolution rate which overcomes the above-described problems in prior art and have additive advantages over them.

Another object of the present invention is to provide a film coated tablet comprising sacubitril in the form of the free base or in the form of pharmaceutically acceptable salts, crystalline polymorph thereof and valsartan in the form of the free base or in the form of pharmaceutically acceptable salts, crystalline polymorph thereof having improved content uniformity and compressibility.

Another object of the present invention is to provide a capsule comprising sacubitril in the form of the free base or in the form of pharmaceutically acceptable salts, crystalline polymorph thereof and valsartan in the form of the free base or in the form of pharmaceutically acceptable salts, crystalline polymorph thereof having improved stability and dissolution profile.

According to this embodiment of the present invention, a solid pharmaceutical composition comprises sacubitril in the form of the free base or in the form of pharmaceutically acceptable salts, crystalline polymorph thereof, valsartan in the form of the free base or in the form of pharmaceutically acceptable salts, crystalline polymorph thereof and at least one pharmaceutically acceptable excipient wherein the amount of binder is 0.1% to 20.0% by weight of the total composition.

According to this embodiment of the present invention, sacubitril is present in the form of sacubitril sodium.

According to this embodiment of the present invention, valsartan is present in the form of valsartan disodium.

According to this embodiment of the present invention, the amount of binder is 1.0 % to 10.0% by weight or 1.0 % to 8.0% by weight of the total composition.

The identification of critical binder attributes in the invention enables rational and efficient binder selection of well soluble and poorly soluble formulations. Binder addition is especially valuable for a poorly soluble formulation. Binder can lead to processing problems such as rapid over granulation, tablet hardness increases and dissolution concert diminish. Especially, in case of some binders, addition of strong disintegrates usually required but these are huge expensive and have a negative effect on product stability.

Suitable binders are selected from the group comprising low-substituted hydroxypropyl cellulose, polyvinylpyrrolidone, hydroxypropyl cellulose, carboxymethylcellulose sodium, cellulose acetate phthalate, chitosan, starch, corn starch, starch mucilage, acacia mucilage, dextrates, dextrin, ethyl cellulose, glyceryl behenate, hydroxyethyl cellulose, hydroxyethylmethyl cellulose, hydroxypropyl starch, hypromellose, magnesium aluminum silicate, methylcellulose, poloxamer, polycarbophil, polydextrose, polyethylene oxide, polymethacrylates, aluminum hydroxide, stearic acid, sucrose, polyoxyethilene-alkyl ethers, pullulan or mixtures thereof.

According to this embodiment of the present invention, cellulose derivatives is used as binders, because cellulosic binders show several interesting characteristics such as low cost, reproducibility, biocompatibility, and recyclability. For example; low-substituted hydroxypropyl cellulose, hydroxypropyl cellulose, hydroxyethyl cellulose, hydroxyethylmethyl cellulose.

According to this embodiment of the present invention, the binder is low-substituted hydroxypropyl cellulose or polyvinylpyrrolidone. Especially, using low-substituted hydroxypropyl cellulose provides the desired dissolution profile and the desired stability.

According to this embodiment of the present invention, the amount of sacubitril sodium is 6.0% to 35.0% by weight of the total composition.

According to this embodiment of the present invention, the amount of sacubitril sodium is 7.0% to 25.0% by weight of the total composition.

According to this embodiment of the present invention, the amount of valsartan disodium is 6.0% to 35.0% by weight of the total composition.

According to this embodiment of the present invention, the amount of valsartan disodium is 10.0% to 25.0% by weight of the total composition.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to this embodiment of the present invention, the pharmaceutically acceptable excipients are selected from the group comprising fillers, disintegrants, pH adjusters, lubricants, glidants or mixtures thereof.

Suitable fillers are selected from group comprising microcrystalline cellulose, pregelatinized starch, mannitol, maltodextrin, dextrin, dextrose, erytritol, fructose, maltose, sucrose, xylitol, dicalcium phosphate, hydroxypropyl betadex, lactose monohydrate, ammonium alginate, calcium carbonate, anhydrous lactose, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, polydextrose, polymethacrylates, sodium alginate, sodium chloride, sorbitol, starch, sugar sphericals, sulfobutylether beta-cyclodextrin, tragacanth, trehalose or mixtures thereof.

According to this embodiment of the present invention, the fillers are selected from group comprising microcrystalline cellulose, pregelatinized starch, mannitol, maltodextrin, dextrin, dextrose, erythritol, fructose, maltose, sucrose, xylitol, dicalcium phosphate, hydroxypropyl betadex or mixtures thereof. The fillers help to provide the desired dissolution profile. Also, it helps to provide the desired stability.

According to this embodiment of the present invention, the filler is microcrystalline cellulose.

According to this embodiment of the present invention, the filler is pregelatinized starch.

According to this embodiment of the present invention, the filler is mannitol, maltodextrin, dextrin, dextrose, erytritol, fructose, maltose, sucrose, xylitol or mixtures thereof.

According to this embodiment of the present invention, the filler is dicalcium phosphate.

According to this embodiment of the present invention, the filler is hydroxypropyl betadex.

According to this embodiment of the present invention, the amount of filler is 25.0% to 75.0% by weight of the total composition.

According to this embodiment of the present invention, the amount of filler is 35.0% to 55.0% by weight or 50.0% to 75.0% by weight of the total composition.

Suitable disintegrants are selected from the group comprising crospovidone, sodium starch glycolate, croscarmellose sodium, carboxymethyl cellulose, sodium alginate, corn starch, alginic acid, magnesium aluminium silica, sodium glycine carbonate or mixtures thereof.

According to this embodiment of the present invention, the disintegrant is crospovidone.

According to this embodiment of the present invention, the amount of disintegrant is 0.5% to 20.0% by weight of the total composition.

According to this embodiment of the present invention, the amount of disintegrant is 2.0% to 10.0% by weight of the total composition.

Suitable lubricants are selected from the group comprising talc, magnesium stearate, sodium stearyl fumarate, calcium stearate, zinc stearate, boric acid, magnesium lauryl sulfate, sodium oleate, polyethylene glycol, stearic acid, glyceryl palmito sulphate, sodium lauryl sulphate or mixtures thereof.

According to this embodiment of the present invention, the lubricant is talc or magnesium stearate or mixtures thereof. It improves the powder processing properties. Also, it enhances powder flow by reducing the inter-particle friction.

According to this embodiment of the present invention, the amount of lubricant is 0.1% to 10.0% by weight of the total composition.

Suitable glidants are selected from group comprising silica colloidal anhydrous, silicon dioxide, aluminium silicate or mixtures thereof.

According to this embodiment of the present invention, the glidant is silica colloidal anhydrous.

According to this embodiment of the present invention, the amount of glidant is 0.1% to 5.0% by weight of the total composition.

When active ingredients have pH-dependent solubility, lower drug absorption and bioavailability are caused. Valsartan is a substance with a very poor water solubility at pH below 5. Above pH 5, both sacubitril and valsartan exhibit high solubility in aqueous medium. The desired dissolution profile is achieved by using suitable pH adjuster.

Suitable pH adjusters are selected from the group comprising disodium phosphate, potassium bicarbonate, potassium citrate, sodium bicarbonate, sodium citrate, aluminum potassium sulfate, anhydrous citric acid, anhydrous disodium hydrogen phosphate, potassium carbonate, anhydrous sodium dihydrogen phosphate, dibasic potassium sulfate, calcium carbonate, nicotinic acid, dilute hydrochloric acid, glacial acetic acid, lactic acid, maleic acid, monobasic potassium phosphate, phosphoric acid, adipic acid, sodium acetate, sodium carbonate, sodium dihydrogen phosphate dihydrate, tartaric acid, tribasic sodium phosphate or mixtures thereof.

According to this embodiment of the present invention, the pH adjuster is disodium phosphate or potassium bicarbonate or potassium citrate or sodium bicarbonate or sodium citrate or mixtures thereof.

According to this embodiment of the present invention, the solid pharmaceutical composition is in the form of a tablet or capsule.

In another embodiment of this present invention, the solid pharmaceutical composition is in the form of a tablet, especially film coated tablet. A film coat on the tablet protects from moisture and further contributes to the ease with which it can be swallowed. Also, it is used in order to minimize for stability problems.

According to this embodiment of the present invention, the solid pharmaceutical composition is in the form of capsule dosage form which comprising mini-capsules or mini-tablets or pellets or granules or powders or mixtures thereof.

An embodiment of this present invention, the form of formulation is pellet in capsule wherein pellet comprises at least one active agent.

According to this embodiment of the present invention, some pellets may be having only sacubitril sodium, other pellets may be having valsartan disodium and then these pellets are mixed. Pellets are located within a capsule.

According to this embodiment of the present invention, active ingredients can be coated on a pellet separately as a separate layer. Then, pellet is located within a capsule.

The solid pharmaceutical composition of the present invention may prepared by direct compression, wet or dry granulation, hot melt granulation, hot melt extrusion, fluidized bed granulation, extrusion/spheronization, slugging, spray drying or solvent evaporation.

According to this embodiment of the present invention, the solid pharmaceutical composition is prepared wet granulation or dry granulation which is simple and cost-effective method. Also, these process helps to provide stability and dissolution profile of the solid pharmaceutical composition.

Solvent is used in the wet granulation. Suitable solvents are selected from the group comprising water, ethanol, dichloromethane, 0.1N HCI, methanol, isopropyl alcohol, benzyl alcohol, propylene glycol, polyethylene glycol, glycerine, cyclomethicone, glycerine triacetate, diethylene glycol monoethyl ether, glycerol formal, propylene carbonate or mixtures thereof.

According to this embodiment of the present invention, the solvent during wet granulation is water or ethanol or mixtures thereof.

According to this embodiment of the present invention, the solid pharmaceutical composition comprises sacubitril sodium, valsartan disodium wherein;
- the amount of low-substituted hydroxypropylcellulose as binder is 1.0% to 10.0% by weight of the total composition,
- Filler is selected from the group comprising mannitol, maltodextrin, dextrin, dextrose, erytritol, fructose, maltose, sucrose, xylitol or mixtures thereof.

According to this embodiment of the present invention, the solid pharmaceutical composition comprises sacubitril sodium, valsartan disodium wherein;
- the amount of binder is 1.0% to 8.0% by weight of the total composition,
- Filler is selected from the group comprising mannitol, maltodextrin, dextrin, dextrose, erytritol, fructose, maltose, sucrose, xylitol or mixtures thereof.

According to this embodiment of the present invention, the solid pharmaceutical composition comprises sacubitril sodium, valsartan disodium wherein;
- the amount of binder is 1.0% to 8.0% by weight of the total composition,
- fillers are selected from group comprising pregelatinized starch, dicalcium phosphate, hydroxypropyl betadex or mixtures thereof.

According to this embodiment of the present invention, the solid pharmaceutical composition comprises sacubitril sodium, valsartan disodium wherein;
- the amount of binder is 1.0% to 8.0% by weight of the total composition,
- Filler is microcrystalline cellulose.

The scope of protection of the invention is set forth in the appended claims and cannot be limited to those described in the detailed description to provide examples. Alternative embodiments which may be carried out by those skilled in the art will constitute a breach of the invention based on the essential aspects of the protection as set forth in the claims.

### Example 1: A tablet comprising sacubitril sodium and valsartan disodium

| **Ingredients** | **% by weight** |
|---|---|
| Sacubitril Sodium | 10.0 - 35.0 |
| Valsartan Disodium | 10.0 - 35.0 |
| Low-substituted hydroxypropylcellulose (L-HPC) | 0.1 -8.0 |
| Microcrystalline cellulose | 25.0 - 70.0 |
| Crospovidone | 0.5 - 15.0 |
| Silica colloidal anhydrous | 0.1 -5.0 |
| Talc | 0.1 -5.0 |
| Magnesium stearate | 0.1 -5.0 |
| **Total Core Tablet** | **100** |
| **Film coating** | |

### Example 2: A tablet comprising sacubitril sodium and valsartan disodium

| **Ingredients** | **% by weight** |
|---|---|
| Sacubitril Sodium | 20.48 |
| Valsartan Disodium | 22.64 |
| Low-substituted hydroxypropylcellulose (L-HPC) | 2.4 |
| Microcrystalline cellulose | 48.68 |
| Crospovidone | 3.2 |
| Silica colloidal anhydrous | 1 |
| Talc | 1 |
| Magnesium stearate | 0.6 |
| **Total Core Tablet** | **100** |
| **Film coating** | |

**A process for example 1 or 2;**
a) Mixing half of L-HPC, half of microcrystalline cellulose and half of crospovidone,
b) Adding magnesium stearate and then mixing,
c) Adding sacubitril sodium and then mixing,
d) Adding valsartan disodium and then mixing,
e) Adding the remaining parts of L-HPC, microcrystalline cellulose and crospovidone and then mixing,
f) Adding talc and then mixing,
g) Compressing to form of tablets,
h) Coating tablets with film coating.

### Example 3: A tablet comprising sacubitril sodium and valsartan disodium

| **Ingredients** | **% by weight** |
|---|---|
| Sacubitril Sodium | 10.0 - 35.0 |
| Valsartan Disodium | 10.0 - 35.0 |
| Low-substituted hydroxypropylcellulose (L-HPC) | 0.1 -8.0 |
| Pregelatinized starch (Low moisture - less than 10% ) | 25.0 - 70.0 |
| Crospovidone | 0.5 - 15.0 |
| Silica colloidal anhydrous | 0.1 -5.0 |
| Talc | 0.1 -5.0 |
| Magnesium stearate | 0.1 -5.0 |
| **Total Core Tablet** | **100** |
| **Film coating** | |

### Example 4: A tablet comprising sacubitril sodium and valsartan disodium

| **Ingredients** | **% by weight** |
|---|---|
| Sacubitril Sodium | 20.48 |
| Valsartan Disodium | 22.64 |
| Low-substituted hydroxypropylcellulose (L-HPC) | 2.4 |
| Pregelatinized starch (Low moisture - less than 10% ) | 48.68 |
| Crospovidone | 3.2 |
| Silica colloidal anhydrous | 1 |
| Talc | 1 |
| Magnesium stearate | 0.6 |
| **Total Core Tablet** | **100** |
| **Film coating** | |

**A process for example 3 or 4;**
a) Mixing half of pregelatinized starch and sacubitril sodium,
b) Adding half of magnesium stearate and then mixing,
c) Mixing the remaining part of pregelatinized starch and valsartan disodium and then mixing,
d) Adding the remaining parts of magnesium stearate and then mixing,
e) Mixing the mixtures at step (b) and step (d),
f) Adding crospovidone and then mixing,
g) Adding silica colloidal anhydrous and then mixing,
h) Adding talc and then mixing,
i) Compressing to form of tablets,
j) Coating tablets with film coating.

### Example 5: A tablet comprising sacubitril sodium and valsartan disodium

| **Ingredients** | **% by weight** |
|---|---|
| Sacubitril Sodium | 10.0 - 35.0 |
| Valsartan Disodium | 10.0 - 35.0 |
| Low-substituted hydroxypropylcellulose (L-HPC) | 0.1 -8.0 |
| Mannitol or maltodextrin or dextrin or dextrose or erythritol or fructose or maltose or sucrose or xylitol | 25.0 - 70.0 |
| Crospovidone | 0.5 - 15.0 |
| Silica colloidal anhydrous | 0.1 -5.0 |
| Talc | 0.1 -5.0 |
| Magnesium stearate | 0.1 -5.0 |
| **Total Core Tablet** | **100** |
| **Film coating** | |

### Example 6: A tablet comprising sacubitril sodium and valsartan disodium

| **Ingredients** | **% by weight** |
|---|---|
| Sacubitril Sodium | 20.48 |
| Valsartan Disodium | 22.64 |
| Low-substituted hydroxypropylcellulose (L-HPC) | 2.4 |
| Mannitol or maltodextrin or dextrin or dextrose or erythritol or fructose or maltose or sucrose or xylitol as a filler | 48.68 |
| Crospovidone | 3.2 |
| Silica colloidal anhydrous | 1 |
| Talc | 1 |
| Magnesium stearate | 0.6 |
| **Total Core Tablet** | **100** |
| **Film coating** | |

**A process for example 5 or 6;**
a) Mixing half of a filler (mannitol or maltodextrin or dextrin or dextrose or erythritol or fructose or maltose or sucrose or xylitol) and sacubitril sodium,
b) Adding half of magnesium stearate and then mixing,
c) Mixing the remaining part of a filler and valsartan disodium and then mixing,
d) Adding the remaining parts of magnesium stearate and then mixing,
e) Mixing the mixtures at step (b) and step (d),
f) Adding crospovidone and then mixing,
g) Adding silica colloidal anhydrous and then mixing,
h) Adding talc and then mixing,
i) Compressing to form of tablets,
j) Coating tablets with film coating.

### Example 7: A tablet comprising sacubitril sodium and valsartan disodium

| **Ingredients** | **% by weight** |
|---|---|
| Sacubitril Sodium | 10.0 - 35.0 |
| Valsartan Disodium | 10.0 - 35.0 |
| Low-substituted hydroxypropylcellulose (L-HPC) | 0.1 -8.0 |
| Dicalcium phasphate | 25.0 - 70.0 |
| Crospovidone | 0.5 - 15.0 |
| Silica colloidal anhydrous | 0.1 -5.0 |
| Talc | 0.1 -5.0 |
| Magnesium stearate | 0.1 -5.0 |
| **Total Core Tablet** | **100** |
| **Film coating** | |

### Example 8: A tablet comprising sacubitril sodium and valsartan disodium

| **Ingredients** | **% by weight** |
|---|---|
| Sacubitril Sodium | 20.48 |
| Valsartan Disodium | 22.64 |
| Low-substituted hydroxypropylcellulose (L-HPC) | 2.4 |
| Dicalcium phasphate | 48.68 |
| Crospovidone | 3.2 |
| Silica colloidal anhydrous | 1 |
| Talc | 1 |
| Magnesium stearate | 0.6 |
| **Total Core Tablet** | **100** |
| **Film coating** | |

**A process for example 7 or 8;**
a) Mixing half of dicalcium phasphate and sacubitril sodium,
b) Adding half of magnesium stearate and then mixing,
c) Mixing the remaining part of dicalcium phasphate and valsartan disodium and then mixing,
d) Adding the remaining parts of magnesium stearate and then mixing,
e) Mixing the mixtures at step (b) and step (d),
f) Adding crospovidone and then mixing,
g) Adding silica colloidal anhydrous and then mixing,
h) Adding talc and then mixing,
i) Compressing to form of tablets,
j) Coating tablets with film coating.

### Example 9; A tablet comprising sacubitril sodium and valsartan disodium

| **Ingredients** | **% by weight** |
|---|---|
| **Inner phase** | |
| Microcrystalline Cellulose | 4.0 - 30.0 |
| Crospovidone | 0.1 - 10.0 |

| **Granulation solution - 1** | |
|---|---|
| PVP K30 | 0.1 - 10.0 |
| Sacubitril Sodium | 6.0 - 25.0 |
| A filler (for example; Hydroxypropyl Betadex) | 10.0 - 40.0 |
| Solvent (water or ethanol or mixture thereof) | q.s. |

| **Granulation solution - 2** | |
|---|---|
| PVP K30 | 0.1 - 10.0 |
| Valsartan Disodium | 6.0 - 25.0 |
| A filler (for example; Hydroxypropyl Betadex) | 10.0 - 40.0 |
| Solvent (water or ethanol or mixture thereof) | q.s. |

| **Outer phase** | |
|---|---|
| Crospovidone | 0.4 - 10.0 |
| Silica colloidal anhydrous | 0.1 -5.0 |
| Talc | 0.1 -5.0 |
| Magnesium stearate | 0.1 -5.0 |
| **Total Core Tablet** | **100** |
| **Film coating** | |

### Example 10; A tablet comprising sacubitril sodium and valsartan disodium

| **Ingredients** | **% by weight** |
|---|---|
| **Inner phase** | |
| Microcrystalline Cellulose | 12.84 |
| Crospovidone | 2.0 |

| **Granulation solution - 1** | |
|---|---|
| PVP K30 | 2.0 |
| Sacubitril Sodium | 10.24 |
| A filler (for example; Hydroxypropyl Betadex) | 26.8 |
| Solvent (water or ethanol or mixture thereof) | q.s. |

| **Granulation solution - 2** | |
|---|---|
| PVP K30 | 2.0 |
| Valsartan Disodium | 11.32 |
| A filler (for example; Hydroxypropyl Betadex) | 26.8 |
| Solvent (water or ethanol or mixture thereof) | q.s. |

| **Outer phase** | |
|---|---|
| Crospovidone | 3.0 |
| Silica colloidal anhydrous | 1.0 |
| Talc | 1.0 |
| Magnesium stearate | 1.0 |
| **Total Core Tablet** | **100** |
| **Film coating** | |

**A process for example 9 or 10;**
**Inner phase;**
   a) Adding microcrystalline cellulose, crospovidone in fluidized bed drying,
**Granulation solution 1**
   b) Mixing pvp K30, sacubitril sodyum, hydroxypropyl betadex and then dissolving in solvent and obtained the granulation solution-1
   c) Spraying the granulation solution-1 at step (b) onto the powder at step (a) in the fluidized bed dryer, and then drying,
**Granulation solution 2**
   d) Mixing pvp K30, valsartan disodyum, hydroxypropyl betadex and then dissolving in solvent and obtained the granulation solution-2
   e) Spraying the granulation solution-2 at step (d) onto the powder at step (c) in the fluidized bed dryer, and then drying,
**Outer phase;**
   f) Adding crospovidone, silica colloidal anhydrous and then mixing,
   g) Adding talc and then mixing,
   h) Adding magnesium stearate and then mixing,
   i) compressing the total mixture into tablets and coating with film coating.

## Claims

1. A solid pharmaceutical composition comprising sacubitril in the form of the free base or in the form of pharmaceutically acceptable salts, crystalline polymorph thereof, valsartan in the form of the free base or in the form of pharmaceutically acceptable salts, crystalline polymorph thereof and at least one pharmaceutically acceptable excipient wherein the amount of binder is 0.1% to 20.0% by weight of the total composition.

2. The solid pharmaceutical composition according to claim 1, wherein sacubitril is present in the form of sacubitril sodium.

3. The solid pharmaceutical composition according to claim 1, wherein valsartan is present in the form of valsartan disodium.

4. The solid pharmaceutical composition according to claim 1, wherein the amount of binder is 1.0 % to 10.0% by weight or 1.0 % to 8.0% by weight of the total composition.

5. The solid pharmaceutical composition according to claim 4, wherein binders are selected from the group comprising low-substituted hydroxypropyl cellulose, polyvinylpyrrolidone, hydroxypropyl cellulose, carboxymethylcellulose sodium, cellulose acetate phthalate, chitosan, starch, corn starch, starch mucilage, acacia mucilage, dextrates, dextrin, ethylcellulose, glyceryl behenate, hydroxyethyl cellulose, hydroxyethylmethyl cellulose, hydroxypropyl starch, hypromellose, magnesium aluminum silicate, methylcellulose, poloxamer, polycarbophil, polydextrose, polyethylene oxide, polymethacrylates, aluminum hydroxide, stearic acid, sucrose, polyoxyethilene-alkyl ethers, pullulan or mixtures thereof.

6. The solid pharmaceutical composition according to claim 4, wherein the binder is low-substituted hydroxypropyl cellulose or polyvinylpyrrolidone.

7. The solid pharmaceutical composition according to claim 1, wherein the pharmaceutically acceptable excipients are selected from the group comprising fillers, disintegrants, pH adjusters, lubricants, glidants or mixtures thereof.

8. The solid pharmaceutical composition according to claim 7, wherein fillers are selected from group comprising microcrystalline cellulose, pregelatinized starch, mannitol, maltodextrin, dextrin, dextrose, erythritol, fructose, maltose, sucrose, xylitol, dicalcium phosphate, hydroxypropyl betadex, lactose monohydrate, ammonium alginate, calcium carbonate, anhydrous lactose, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, polydextrose, polymethacrylates, sodium alginate, sodium chloride, sorbitol, starch, sugar sphericals, sulfobutylether beta-cyclodextrin, tragacanth, trehalose or mixtures thereof.

9. The solid pharmaceutical composition according to claim 7, wherein fillers are selected from group comprising microcrystalline cellulose, pregelatinized starch, mannitol, maltodextrin, dextrin, dextrose, erythritol, fructose, maltose, sucrose, xylitol, dicalcium phosphate, hydroxypropyl betadex or mixtures thereof.

10. The solid pharmaceutical composition according to claim 1, wherein the solid pharmaceutical composition is in the form of a tablet or capsule.

11. The solid pharmaceutical composition according to claim 11, wherein the form of composition is in the form of a tablet.

12. The solid pharmaceutical composition according to claim 10, wherein the composition is in the form of capsule dosage form which comprising mini-capsules or mini-tablets or pellets or granules or powders or mixtures thereof.

13. The solid pharmaceutical composition according to claim 11, wherein the form of composition is pellet in capsule wherein pellet comprising at least one active agent.

14. The solid pharmaceutical composition according to claim 11, wherein active ingredients is coated on a pellet separately as a separate layer.
